# EUROPÄISCHE PATENTSCHRIFT

(11) **EP 1 074 294 B1**
(45) Veröffentlichungstag und Bekanntmachung des Hinweises auf die Patenterteilung: **09.11.2005**
(21) Anmeldenummer: 00116165.2
(22) Anmeldetag: 01.08.2000
(51) Int. Cl.: B01J 10/00, B01J 35/06

(54) **Vorrichtung und Verfahren zur isothermen Durchführung einer Dreiphasenhydrierung von Benzol zu Cyclohexan**
Device and method for isothermally carrying out a three-phase hydrogenation of benzene to cyclohexane
Dispositif et procédé pour la mise en oeuvre isothermique de la hydrogénation tri-phasiques de benzène à cyclohexane

(30) Priorität: 02.08.1999 DE 19936276
(43) Veröffentlichungstag der Anmeldung: 07.02.2001
(73) Patentinhaber: BASF Aktiengesellschaft, 67056 Ludwigshafen (DE)
(72) Erfinder: Bröcker, Franz Josef, Dr., 67061 Ludwigshafen (DE); Haake, Mathias, Dr., 68161 Mannheim (DE); Kaibel, Gerd, Dr., 68623 Lampertheim (DE); Rohrbacher, Gerd, 67069 Ludwigshafen (DE); Schwab, Ekkehard, Dr., 67434 Neustadt (DE); Stroezel, Manfred, 68549 Ilvesheim (DE)
(74) Vertreter: Isenbruck, Günter

(56) Entgegenhaltungen:
- EP-A- 0 798 039
- DD-A- 226 872
- DE-A- 19 641 707
- US-A- 4 514 520
- US-A- 5 773 670

## Beschreibung

Die Erfindung betrifft ein Verfahren und eine Vorrichtung zur isothermen Durchführung eines Hydrierung von Benzol zu Cyclohexan, bei der mindestens drei Phasen in Form einer gasförmigen, einer flüssigen und einer festen Phase beteiligt sind.

Die Erfindung betrifft speziell die Durchführung solcher Reaktionen, bei denen zumindest ein Edukt flüssig und eines gasförmig ist und bei denen Feststoffkatalyse vorliegt.

Die Durchführung solcher Reaktionen ist mit erheblichen Schwierigkeiten verbunden. Der Stoffübergang Gas/Flüssigkeit ist oft problematisch. Auch sind isotherme Bedingungen nur schwer zu erreichen. Isotherm bedeutet, daß die Reaktionswärme durch Zu- oder Abführen von Wärme weitgehend ausgeglichen wird, so daß zeitliche oder örtliche Temperaturschwankungen im Reaktor nicht ins Gewicht fallen.
Bisher haben sich die in G. Eigenberger, Ullmann, 5. Auflage, Bd. 4, Seite 199ff. (1992) Wiley-VCH, Weinheim, Berlin, New-York beschriebenen Verfahren durchgesetzt.

In EP-B 0 305 203 (US-A 4 985 230) wird die Durchführung heterogen katalysierter Reaktionen unter nicht adiabatischen Bedingungen beschrieben. Dazu werden monolithische Katalysatoren in einen Reaktor gebracht, der wärmeabführende Wände besitzt. Ein monolithischer Katalysator ist ein zusammenhängender Festkörper mit katalytischer Oberfläche, die so groß ist, daß abzählbare Mengen dieser Körper ausreichen, um die betreffende Reaktion in technisch sinnvollem Umfang zu katalysieren. Die monolithischen Katalysatoren weisen Kanäle auf, die schräg zur Hauptströmungsrichtung stehen, so daß das Reaktionsfluid im spitzen Winkel von einer Reaktorwand zur anderen geleitet wird, was den Wärmetransport verbessern soll. Der Scherstreß, der auf das Reaktionsfluid ausgeübt wird, ist in der Nähe der Reaktorwand extrem hoch (hoher Druckverlust) und ansonsten eher niedrig (schlechter Stofftransport). Dies führt zu unnötig großen Druckverlusten in Wandnähe. Die Fertigungsweise des Reaktors ist aufwendig, da der Druckverlust entscheidend von der Geometrie zwischen Reaktorwand und monolithischem Katalysator abhängt.

In EP-B 0 201 614 (US-A 4 731 229) wird ein Reaktor beschrieben, der bandförmige teilweise gewellte Katalysatorköper enthält, deren Wellung schräg zur Hauptströmungsachse geneigt und bei benachbarten Platten entgegengesetzt gerichtet ist, wobei die Wellenlänge der Wellung des Katalysatorkörpers kleiner als die Wellenlänge der benachbarten gewellten Platten ist und die Oberfläche des Katalysatorkörpers größer ist als die Oberfläche einer benachbarten gewellten Platte. Bei dieser Vorrichtung ist die Erzeugung von Gas-Füssigkeits-Dispersionen nicht vorgesehen. Die komplizierte Wellung der Platten begünstigt Bypass-Bildung verhindert Wirbelbildung und erschwert somit den Stofftransport. Außerdem ermöglicht das vorgesehene kompakte Pachungselement keine effektive Abfuhr der Reaktionswärme.

Aus EP-B 0 068 862 (CA-A 1 146 148) ist ein Festbettreaktor für Transferreaktionen zwischen Gasphase und Flüssigkeit bekannt. Bei diesem Reaktor besteht das Festbett aus alternierenden Lagen von ebenen und gewellten Bahnen, die zu einer Rolle aufgewickelt sind, wobei die gewellte Bahn aus offenmaschigem Material mit zumindest einer äußeren Oberflächenschicht besteht, die eine organische polymere Substanz hohen Molekulargewichts aufweist, die inhärent hydrophob bezüglich der genannten Flüssigkeit ist, und wobei die ebene Bahn aus gewelltem, gewebtem oder gefilztem Tuch eines textilen dochtartigen Materials besteht, das hydrophil bezüglich der Flüssigkeit der Gas-Flüssigkeit-Transferreaktion ist und das einen ununterbrochenen Dochtpfad zwischen den Enden der Rolle für die genannte Flüssigkeit bildet. Nachteilig ist bei diesem Reaktortyp, daß die textilen Bestandteile des Reaktors die Querschnittsbelastungen begrenzen. Der Dochtpfad behindert außerdem einen schnellen Flüssigkeitsaustausch, begünstigt somit die Entmischung zwischen Gas und Flüssigkeit und behindert den Stofftransport zwischen Gas und Flüssigkeit. Im übrigen ist der Reaktor für den adiabatischen Betrieb vorgesehen.

Aus EP-A 0 798 039 sind ein Verfahren und ein Reaktor zur Durchführung von Stoffumwandlungen mit in Flüssigkeiten suspendierten Katalysatoren bekannt. Im Reaktor kann eine Schüttung, ein Gestrick, eine offenzellige Formstruktur oder ein Packungselement aufgenommen sein. Weiterhin kann der Reaktor heiz- oder kühlbar sein. Als geeignete Reaktoren werden z.B. Rohrbündelreaktoren, Spiral- oder Plattenwärmetauscher genannt.

DE-A 196 41 707 hat ein Verfahren zur Herstellung von 1,4-Butandiol durch katalytische Hydrierung von 1,4-Butindiol zum Gegenstand. Die Reaktion wird in Gegenwart eines im Reaktionsmedium suspendierten Katalysators durchgeführt. Es wird weiterhin erwähnt, dass auch mit katalytisch aktiven Komponenten beschichtete Packungen im Reaktor eingesetzt werden können.

Es ist somit Aufgabe der Erfindung, eine Vorrichtung und ein Verfahren zur Durchführung eines Hydrierung von Benzol zu Cyclohexan unter, Beteiligung einer flüssigen, einer gasförmigen und einer festen Phase bereitzustellen, die einen verbesserten Stofftransport zwischen Gasphase und Flüssigkeit und eine isotherme Reaktionsführung ermöglichen.

Die Aufgabe wird gelöst durch eine Vorrichtung zur Durchführung eines Hydrierung von Benzol zu Cyclohexan unter Beteiligung einer gasförmigen, Wasserstoff enthaltenden Phase, einer flüssigen, Benzol enthaltenden Phase und einer festen, Katalysator enthaltenden Phase, aufweisend
- ein Dispersionsorgan, das zur Erzeugung eines Reaktionsfluids durch Dispersion einer Gasphase in einer flüssigen Phase ausgestaltet ist,
- mindestens einen Reaktor mit Einlaß und Auslaß, wobei der Reaktorraum durch wärmeabführende Wände begrenzt ist, die einen im wesentlichen konstanten Abstand längs der Hauptströmungsachse des Reaktionsfluids aufweisen, und der Reaktor mit Katalysator beschichtetem Metallflechtwerk bestückt ist und
- eine Zuleitung, die das Reaktionsfluid vom Dispersionsorgan zum Reaktoreinlaß leitet und so kurz ist, daß der Dispersitätsgrad des Reaktionsfluids sich bei der Passage der Zuleitung im wesentlichen nicht ändert.

Der Erfindung liegt die Erkenntnis zugrunde, daß ein verbesserter Stofftransport nur gewährleistet werden kann, wenn als Reaktionsfluid eine Dispersion aus Gasphase (Dispergens) und Flüssigkeit (Dispersionsmittel) hergestellt wird und Verfahren und Vorrichtung so ausgestaltet werden, daß die Dispersion bei dem Durchtritt durch den Reaktor stabil bleibt, d. h. im wesentlichen kein Anstieg der Blasengröße eintritt.

Der erfindungsgemäße Reaktor ist zur Aufrechterhaltung einer hohen aber gleichmäßigen Scherbelastung des Reaktionsfluides ausgestaltet. Zum einen hält er einer hohen Querschnittsbelastung stand, ohne daß es zur Zermahlung des Katalysators kommt. Zum anderen wird das Reaktionsfluid in dem Metallflechtwerk einer gleichmäßig hohen Scherbelastung ausgesetzt. Hierdurch kommt es zu einer gleichmäßigen Durchwirbelung des Reaktionsfluids und damit zu einer Konstanthaltung des Dispersitätsgrades des Reaktionsfluids bei der Passage durch den Reaktor.

Bei dem erfindungsgemäßen mit Katalysator beschichteten Metallflechtwerk handelt es sich um Metallgewebe oder Metallgestricke. Der Drahtdurchmesser beträgt im allgemeinen 0,01 bis 5,0 mm, bevorzugt 0,04 bis 1,0 mm. Die Maschenweite kann über einen weiten Bereich variiert werden.

Diese Gewebe oder -getricke können nach dem in EP-B 0 564 830 (CA-A 2 090 930) oder in EP-A 0 965 384 beschriebenen Verfahren beschichtet werden. Die Beschichtung von Metallgestricken mit Katalysator ist in EP-B 0 564 830 nicht explizit beschrieben, es ist jedoch wie bei Metallgeweben zu verfahren. Im Rahmen der Erfindung wird unter Metallgestricken ein Flechtwerk aus Metall verstanden, das durch einen umlaufenden Metallfaden gebildet wird. Unter Metallgewebe hingegen wird ein Flechtwerk aus mindestens zwei Metallfäden verstanden.

Die Beschichtung von Metallgeweben oder -gestricken mit Katalysatoren kann auch durch herkömmliche Tauchverfahren erfolgen, z.B. nach dem in EP-A 0 056 435 beschriebenen Verfahren.

Ist das Metall, aus welchem das Metallgewebe oder -gestrick besteht (eventuell nach einer Behandlung) selbst katalytisch aktiv, so kann auf eine Beschichtung gänzlich verzichtet werden.

Metallgewebe oder -gestricke können in Form von Bändern verwandt werden. Durch Verformung mittels einer Zahnradwalze können die mit Katalysator beschichteten Metallgewebe oder -gestricke gewellt werden. Das Einbringen von gewelltem Metallgewebe oder -gestrick in den Reaktor ermöglicht es, die Pakkungsdichte des Metallgewebes oder -gestricks zu verändern. Es können zum Beispiel mehrere Lagen aus gewelltem und glattem Metallgewebe oder -gestrick in den Reaktorraum eingebracht werden. Auch das Einbringen von inerten Blechen zwischen Lagen aus Metallgewebe und -gestrick ist möglich. Das Einbringen der mit Katalysator beschichteten Metallgewebe oder -gestricke hat jedenfalls so zu erfolgen, daß der Reaktorraum zwischen den wärmeableitenden Wänden möglichst gleichmäßig gefüllt ist. Die gleichmäßige Füllung unterdrückt Bypass-bildung und unterstützt die Wärmeleitung zu den wärmeabführenden Reaktorwänden, die ihrerseits eine isotherme Reaktionsführung ermöglichen.

In einer weiteren Ausführungsform ist das Dispersionsorgan ein Flüssigkeitsstrahl-Gasverdichter. Bei diesen per se bekannten Dispersionsvorrichtungen handelt es sich um Strahlpumpen zum Fördern und Verdichten von Gasen.

Ihre Wirkung beruht darauf, daß der Treibflüssigkeitsstrahl nach dem Austritt aus der Treibdüse in einzelne Tropfen aufgelöst wird. Diese verteilen sich gleichmä-ßig über den Querschnitt der Mischdüse, nehmen durch Stoß und Reibung die umgebenden Gasteile mit und verdichten sie auf einen höheren Druck. Der erreichbare Dispersitätsgrad wird bestimmt durch die Abstimmung von Treibdüse und Defusor. Diese wiederum hängt vom Treibflüssigkeitsdruck, dem Saugdruck, dem Gegendruck, dem Treibflüssigkeitsstrom, dem Gassaugstrom und dem Gemischstrom ab.

In einer weiteren Ausführungsform der erfindungsgemäßen Vorrichtung ist der Reaktor als Wärmetauscher ausgebildet. Der Wärmedurchgang durch die Reaktorwand wird entscheidend beschleunigt, wenn auf der dem Reaktorraum abgewandten Seite der Reaktorwand ein strömendes Fluid die Reaktionswärme aufnimmt und abtransportiert. Ein solcher Wärmetauscher-Reaktor kann als Plattenerwärmetauscher oder als Spiralwärmetauscher ausgebildet sein. Ein erfindungsgemäßer Plattenwärmetauscher-Reaktor weist einen insbesondere quadratischen oder rechteckigen Reaktorraum auf, der durch zusätzliche wärmeableitende Wände unterteilt ist, die dem Reaktionsfluid einen zickzackförmigen Kurs durch den Reaktorraum aufzwingt. An den Stellen der größten Richtungsänderung wird auf Katalysator beschichtetes Metallgewebe oder -gestrick verzichtet, um einen zu starken Druckabfall zu vermeiden. Ein erfindungsgemäßer Spiralwärmetauscher-Reaktor weist einen insbesondere zylindrischen Reaktorraum auf, der möglichst gleichmäßig mit mit Katalysator beschichteten Metallgeweben oder -gestricken bestückt ist. Der Wandabstand der erfindungsgemäßen Wärmetauscher-Reaktoren beträgt vorzugsweise 1 bis 30 mm, vor allem 2 bis 20 mm, insbesondere 4 bis 10 mm.

Ein weiterer Gegenstand der Erfindung ist ein Verfahren zur Durchführung von Reaktionen unter Beteiligung einer gasförmigen, einer flüssigen und einer festen Phase gekennzeichnet durch die folgenden Schritte:
- Erzeugung eines Reaktionsfluids durch Dispersion einer Gasphase in einer flüssigen Phase,
- Durchleiten des erzeugten Reaktionsfluids durch einen Reaktor, dessen Reaktorraum mit Metallgeweben oder -gestricken bestückt ist, die mit Katalysator beschichtet worden sind,
- Austausch der Reaktionswärme an den Wänden, die den Reaktorraum begrenzen und
- Auftrennen des Reaktionsfluids in Gasphase und flüssige Phase.
Die Auftrennung des Reaktionsfluids kann mit herkömmlichen Abscheidern vorgenommen werden.

Das Verfahren wird bevorzugt in Sumpffahrweise durchgeführt, d.h. die Hauptströmungsrichtung des Reaktionsfluids im Reaktor zeigt nach oben.

Eine weitere Ausführungsform des erfindungsgemäßen Verfahrens ist gekennzeichnet durch die getrennte teilweise Rückführung von Gasphase und/oder flüssiger Phase. Unter getrennter teilweiser Rückführung wird verstanden, daß das Reaktionsprodukt aus der Gasphase und/oder aus der flüssigen Phase abgetrennt wird. Das verbleibende Gas und die verbleibende Flüssigkeit können vollständig oder zum Teil erneut dispergiert und dem Reaktor zugeführt werden.

In einer weiteren Ausführungsform des erfindungsgemäßen Verfahrens beträgt die Flüssigkeitsbelastung des Reaktors 100 bis 600 m³/(m² · h), vorzugsweise 150 bis 300 m³/(m² · h), bezogen auf den leeren Reaktorquerschnitt senkrecht zur Hauptströmungsachse. Unter Flüssigkeitsbelastung, bezogen auf den leeren Reaktorquerschnitt, wird der bei den Reaktionsbedingungen (Druck und Temperatur) vorliegende Volumenstrom des Flüssigkeitsanteils der Dispersion dividiert durch die Querschnittsfläche des Reaktorraums senkrecht zur Hauptströmungsachse verstanden. Da durch das Einbringen von Metallgeweben oder -gestricken dem Reaktionsfluid nicht der gesamte Reaktorraum zur Verfügung steht, ist die tatsächliche mikroskopische Flüssigkeitsbelastung, bezogen auf den lichten Reaktorquerschnitt, entsprechend höher.

In einer weiteren Ausführungsform des erfindungsgemäßen Verfahrens beträgt die Gasbelastung 0,5 bis 15 cm/s, vorzugsweise 2,5 bis 10 cm/s, bezogen auf den leeren Reaktorquerschnitt senkrecht zur Hauptströmungsachse. Die Gasbelastung wird im Rahmen der Erfindung analog der Flüssigkeitsbelastung definiert.

In einer weiteren Ausführungsform des erfindungsgemäßen Verfahrens steht das Reaktionsfluid im Reaktor unter einem Druck von 0,1 bis 200 bar, vorzugsweise 1 bis 100 bar, insbesondere 1 bis 10 bar.

In einer weiteren Ausführungsform des erfindungsgemäßen Verfahrens weist das Reaktionsfluid im Reaktor eine Temperatur von 25 bis 250°C, vorzugsweise 25 bis 200°C, insbesondere 50 bis 150°C auf.

Die Erfindung wird im folgenden anhand der Figuren 1 bis 4 näher beschrieben. Es zeigt
Fig. 1 eine Vorrichtung für eine Dreiphasenreaktion mit Produktrückführung, Kreisgasfahrweise unter Verwendung eines Flüssigkeitsstrahl-Gasverdichters und eines Plattenwärmetauscher-Reaktors.
Fig. 2 eine Vorrichtung für eine Dreiphasenreaktion mit Produktrückführung, Kreisgasfahrweise unter Verwendung eines Flüssigkeitsstrahl-Gasverdichters und eines Spiralwärmetauscher-Reaktors.
Fig. 3 Seitenaufsicht auf das Innere eines Spiralwärmetauscher-Reaktors.
Fig. 4 Seitenansicht auf einen Spiralwärmetauscher-Reaktor.

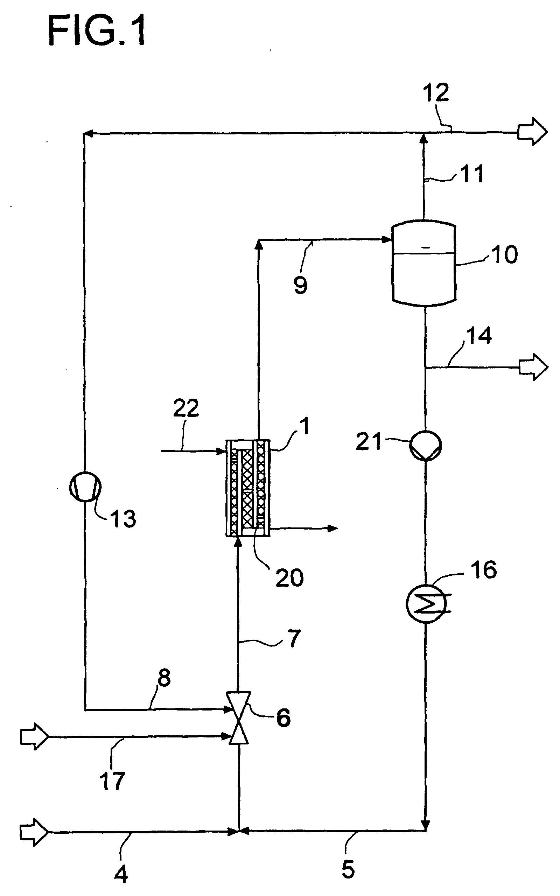

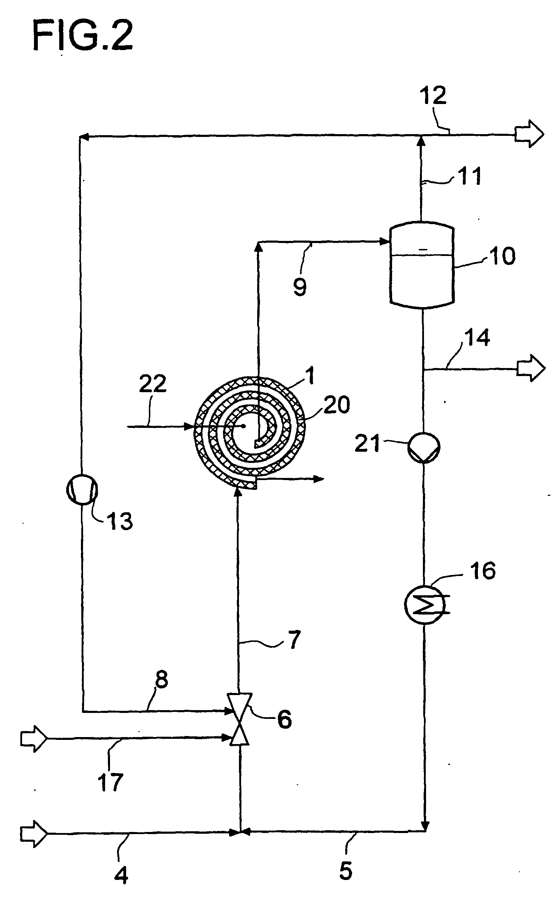

Die in Fig. 1 und 2 beschriebene Vorrichtung wird nach der Aktivierung des auf dem Metallflechtwerk **20** befindlichen Katalysators im Reaktor **1** (z.B. durch Reduktion mit H₂) mit Produktflüssigkeit gefüllt. Hierzu wird mit Hilfe der Kreislaufpumpe **21** die Flüssigkeit vom Abscheider **10** über den optionalen Vorheizer **16** und die Zuleitung zum Flüssigkeitsstrahl-Gasverdichter **5** zum Flüssigstrahl-Gasverdichter **6** gepumpt und von dort in den Wärmetauscher-Reaktor **1** und von diesem über die Zuleitung zum Abscheider **9** in den Abscheider **10** zurück. Kreisgas wird über die Zuleitung **11** aus dem Abscheider **10** abgezogen und mittels der Kreisgaspumpe **13** über die Zuleitung zum Flüssigkeitsstrahl-Gasverdichter **8** zum Flüssigkeitsstrahl-Gasverdichter **6** geführt. Hier erfolgt eine Verdichtung des Gases unter gleichzeitiger Dispersion in der Flüssigkeit unter Bildung des Reaktionsgemisches. Durch eine Zuleitung zum Reaktor **7**, die so kurz sein muß, daß sich entlang dieser Wegstrecke der Dispersitätsgrad des Reaktionsfluids sich im wesentlichen nicht ändert, wird das Reaktionsfluid in den Reaktor **1** geführt. Ist der Kreislauf mit Produkt angefahren, so wird über die Zuleitung zum Flüssigkeitsstrahl-Gasverdichter **4** Edukt zugefahren, und über eine Standhaltung des Abscheiders **10** die entsprechende Menge Produkt über die Ableitung **14** dem Flüssigkeitskreislauf entzogen. Frischgas, welches den Verbrauch an Reaktionsgas ersetzt, wird durch die Zuleitung zum Flüssigkeitsstrahl-Gasverdichter **17** unter Aufrechterhaltung des Drucks in den Gaskreislauf eingespeist und Abgas über die Abgasleitung **12** dem Gaskreislauf entzogen. Die Reaktionswärme wird bei exothermen Reaktionen über den Kühlkreislauf **22** aus dem Reaktor abgerührt, bzw. bei endothermen Reaktionen zugeführt.

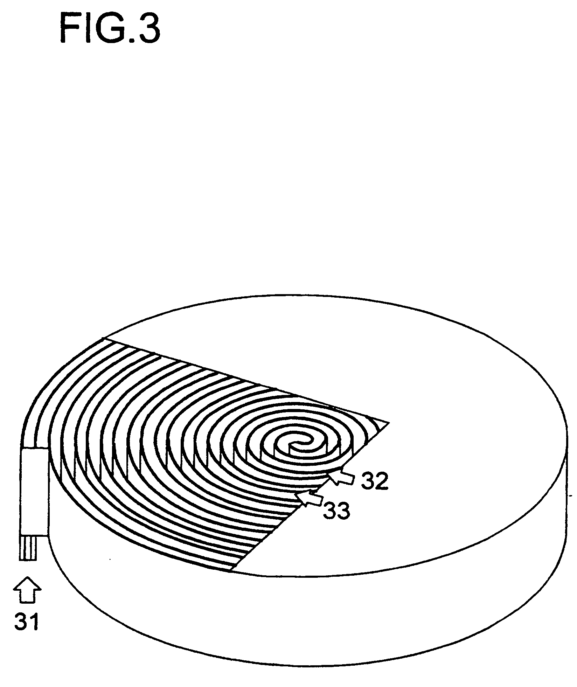

Fig. 3 zeigt eine Seitenansicht auf einen erfindungsgemäßen Spiralwkmetauscher-Reaktor. **31** bezeichnet den Zulauf des Reaktionsfluids in den Reaktor (Reaktoreinlaß). **32** bezeichnet den Reaktorspalt, der das Katalysator-beschichtete Metallflechtwerk aufnimmt, das in mehr oder weniger dichter Packung den gesamten Raum ausfüllt. **33** bezeichnet den Kühlspalt, der das Kühlfluid aufnehmen soll.

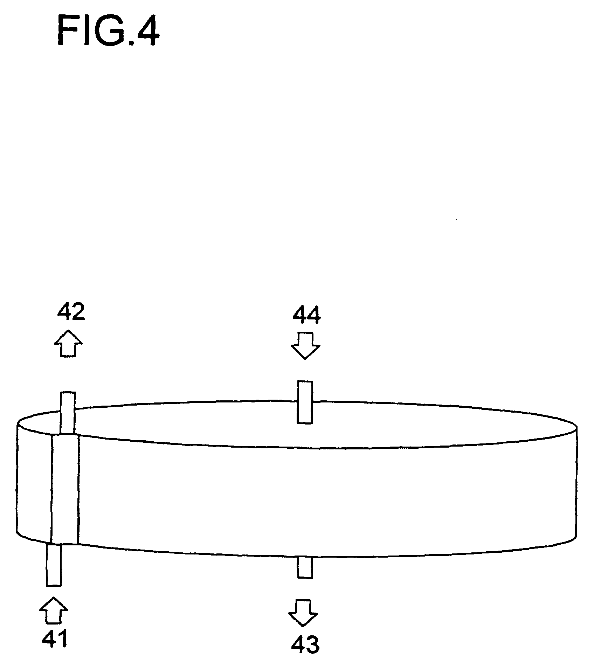

Fig. 4 stellt eine Seitenansicht auf einen Spiralwärmetauscher-Reaktor dar und bezeichnet die Anordnung der Zulauf- und Ablaufstutzen. **41**: Zulauf Reaktionsfluid (Reaktoreinlaß), **42:** Ablauf Kühlfluid, **43:** Ablauf Reaktionsfluid (Reaktorauslaß), **44:** Zulauf Kühlfluid. Reaktionsfluid und Kühlfluid sind hier im Gegenstrom angeordnet, um maximale Wärmemengen auszutauschen. Ist gerade die am Reaktoreintritt freigesetzte Wärmemenge in Hinblick auf z.B. Selektivität und Katalysatorstabilität kritisch, dann ist eine Anordnung im Gleichstromprinzip empfehlenswert.

Das nachfolgende Beispiel erläutert die Erfindung zusätzlich.

### Beispiel

Die Hydrierung von Benzol zu Cyclohexan weist eine Wärmetönung von Δ*H* = -214*kJ* / *mol* auf.
Bei der Benzolhydrierung kann eine Gleichgewichtseinstellung zwischen Cyclohexan und Methylcyclopentan nur vermieden werden, wenn die Reaktionswärme abgeführt und eine relativ tiefe Temperatur eingehalten wird.
Untersuchungen haben ferner gezeigt, daß die Reaktion durch geringe Löslichkeit von Wasserstoff in Benzol und Cyclohexan stofflimitiert ist, d.h. daß das Reaktionsgemisch längs der Katalysatorschicht an gelöstem H₂ verarmt. Es ist deshalb vorteilhaft, das Nachlösen von Wasserstoff erfindungsgemäß zu verbessern.
Mit einer erfindungsgemäßen Vorrichtung gemäß Fig. 2, enthaltend einen Spiralwärmetauscher-Reaktor gemäß Figuren 3 und 4, wurde die Benzolhydrierung durchgeführt. Dazu wurde der Reaktorspalt von 5 mm Breite, 25 mm Tiefe und 960 mm Länge (Volumen 120 ml) mit 8 Lagen Katalysatorgestrickband gefüllt. Zur Herstellung des Katalysatorgestrickbands wurde zunächst ein V2A-Trägerstrickband (Werkstoffnummer 1.4301) 3 h bei 650 °C an der Luft getempert und anschließend im Vakuum mit 6 nm Pt beschichtet. Die Menge an Aktivkomponente betrug 46 mg. Der so mit Katalysator bestückte Wärmetauscher-Reaktor wurde in die in Fig. 3 dargestellte Apparatur eingebaut. Nach Spülung mit Stickstoff und Reduktion des Katalysators bei 80°C über 2 h mit Wasserstoff, wurde über die Zuleitung **4** Benzol in den mit Cyclohexan gefüllten Flüssigkeitskreislauf gepumpt. Die Reaktionsparameter betrugen p = 20 bar, T = 90 °C mit einer auf den leeren Reaktorquerschnitt bezogenen Querschnittsbelastung von Flüssigkeit und Wasserstoff von 400 m³/m²h.
Am Reaktorausgang wurde die Temperatur des Reaktionsprodukts gemessen. Es ergab sich eine maximale Temperaturdifferenz von 0,2 °C bezogen auf die eingestellte Reaktionstemperatur.
Bei einem Umsatz von 98 % wurde eine Selektivität von 100 % erzielt. Die Raum-Zeit-Ausbeute bezogen auf das Volumen des Reaktorspalts betrug 0,5 kg/(l·h).

## Patentansprüche

1. Vorrichtung zur Durchführung einer Hydrierung von Benzol zu Cyclohexan unter Beteiligung einer gasförmigen, Wasserstoff enthaltenden Phase, einer flüssigen, Benzol enthaltenden Phase und einer festen, Katalysator enthaltenden Phase, aufweisend
- ein Dispersionsorgan (6), das zur Erzeugung eines Reaktionsfluids durch Dispersion einer Gasphase in einer flüssigen Phase ausgestaltet ist,
- mindestens einen als Wärmetauscher ausgebildeten Reaktor (1) mit Einlaß (31, 41) und Auslaß (43), wobei der Reaktorraum durch wärmeabführende Wände begrenzt ist, die einen im wesentlichen konstanten Abstand längs der Hauptströmungsachse des Reaktionsfluids aufweisen, und der Reaktor mit Katalysator beschichtetem Metallflechtwerk (20, 32) bestückt ist und
- eine Zuleitung (7), die das Reaktionsfluid vom Dispersionsorgan (6) zum Reaktoreinlaß (31, 41) leitet und so kurz ist, daß der Dispersitätsgrad des Reaktionsfluids sich bei der Passage der Zuleitung im wesentlichen nicht ändert.

2. Vorrichtung nach Anspruch 1, **dadurch gekennzeichnet, daß** das Metallflechtwerk (20, 32) Metallgewebe ist.

3. Vorrichtung nach Anspruch 1, **dadurch gekennzeichnet, daß** das Metallflechtwerk (20, 32) Metallgestrick ist.

4. Vorrichtung nach einem der Ansprüche 1 bis 3, **dadurch gekennzeichnet, daß** das Dispersionsorgan (6) ein Flüssigkeitsstrahl-Gasverdichter ist.

5. Vorrichtung nach einem der Ansprüche 1 bis 4, **dadurch gekennzeichnet, daß** der Reaktor (1) als Plattenwärmetauscher ausgebildet ist.

6. Vorrichtung nach einem der Ansprüche 1 bis 4, **dadurch gekennzeichnet, daß** der Reaktor (1) als Spiralwärmetauscher ausgebildet ist.

7. Vorrichtung nach einem der Ansprüche 1 bis 6, **dadurch gekennzeichnet, daß** der Wandabstand im Reaktor 1 bis 30 mm beträgt.

8. Vorrichtung nach einem der Ansprüche 1 bis 6, **dadurch gekennzeichnet, daß** der Wandabstand im Reaktor 2 bis 20 mm beträgt.

9. Vorrichtung nach einem der Ansprüche 1 bis 6, **dadurch gekennzeichnet, daß** der Wandabstand im Reaktor 4 bis 10 mm beträgt.

10. Verfahren zur Durchführungeiner Hydrierung von Benzol zu Cyclohexan unter Beteiligung einer gasförmigen, Wasserstoff enthaltende Phase, einer flüssigen, Benzol enthaltenden Phase und einer festen, Katalysator enthaltenden Phase, **gekennzeichnet durch** die folgenden Schritte
- Erzeugung eines Reaktionsfluids **durch** Dispersion einer Gasphase in einer flüssigen Phase,
- Durchleiten des erzeugten Reaktionsfluids **durch** einen Reaktor, dessen Reaktorraum mit Metallgeweben oder -gestricken bestückt ist, die mit Katalysator beschichtet worden sind,
- Austausch der Reaktionswärme an den Wänden, die den Reaktorraum begrenzen, und
- Auftrennen des Reaktionsfluids in Gasphase und flüssige Phase.

11. Verfahren nach Anspruch 10, **gekennzeichnet durch** die getrennte teilweise Rückführung von Gasphase und/oder flüssiger Phase.

12. Verfahren nach Anspruch 10, **dadurch gekennzeichnet, daß** die Flüssigkeitsbelastung des Reaktors 100 bis 600 m³/(m² · h), bezogen auf den leeren Reaktorquerschnitt senkrecht zur Hauptströmungsachse, beträgt.

13. Verfahren nach Anspruch 10, **dadurch gekennzeichnet, daß** die Gasbelastung 0,5 bis 15 cm/s, bezogen auf den leeren Reaktorquerschnitt senkrecht zur Hauptströmungsachse, beträgt.

14. Verfahren nach Anspruch 10, **dadurch gekennzeichnet, daß** das Reaktionsfluid im Reaktor unter einem Druck von 0,1 bis 200 bar steht.

15. Verfahren nach Anspruch 10, **dadurch gekennzeichnet, daß** das Reaktionsfluid im Reaktor eine Temperatur von 25 bis 250°C aufweist.

## Claims

1. Apparatus for carrying out a hydrogenation of benzene to cyclohexane involving a gaseous hydrogen-comprising phase, a liquid benzene-comprising phase and a solid catalyst-comprising phase, comprising
- a dispersing element (6) for dispersing a gas phase in a liquid phase to generate a reaction fluid,
- at least one reactor (1) which is constructed as a heat exchanger, which possesses an inlet (31, 41), an outlet (43) and a reactor space bounded by heat-removing walls which are spaced apart substantially uniformly along the main flow axis of the reaction fluid, and which is fitted with catalyst-coated metal fabric (20, 32), and
- a feed line (7) which routes the reaction fluid from the dispersing element (6) to the reactor inlet (31, 41) and is sufficiently short that the degree of dispersion of the reaction fluid does not substantially change in the course of the passage through the feed line.

2. Apparatus as claimed in claim 1, wherein the metal fabric (20, 32) is woven metal fabric.

3. Apparatus as claimed in claim 1, wherein the metal fabric (20, 32) is knitted metal fabric.

4. Apparatus as claimed in any of claims 1 to 3, wherein the dispersing element (6) is a liquid jet gas compressor.

5. Apparatus as claimed in any of claims 1 to 4, wherein the reactor (1) is constructed as a plate type heat exchanger.

6. Apparatus as claimed in any of claims 1 to 4, wherein the reactor (1) is constructed as a spiral type heat exchanger.

7. Apparatus as claimed in any of claims 1 to 6, wherein the walls in the reactor are spaced from 1 to 30 mm apart.

8. Apparatus as claimed in any of claims 1 to 6, wherein the walls in the reactor are spaced from 2 to 20 mm apart.

9. Apparatus as claimed in any of claims 1 to 6, wherein the walls in the reactor are spaced from 4 to 10 mm apart.

10. A process for carrying out a hydrogenation of benzene to cyclohexane involving a gaseous hydrogen-comprising phase, a liquid benzene-comprising phase and a solid catalyst-comprising phase, which comprises the steps of
- generating a reaction fluid by dispersing a gas phase in a liquid phase,
- passing the generated reaction fluid through a reactor whose reactor space is equipped with woven or knitted metal fabrics coated with catalyst,
- transferring the heat of reaction at the walls which bound the reactor space, and
- separating the reaction fluid into gas phase and liquid phase.

11. A process as claimed in claim 10, operated with separate partial recycling of gas phase and/or liquid phase.

12. A process as claimed in claim 10, wherein the superficial liquid velocity in the reactor is from 100 to 600 m³/(m²·h).

13. A process as claimed in claim 10, wherein the superficial gas velocity is from 0.5 to 15 cm/s.

14. A process as claimed in claim 10, wherein the reaction fluid in the reactor is under a pressure of from 0.1 to 200 bar.

15. A process as claimed in claim 10, wherein the reaction fluid in the reactor has a temperature of from 25 to 250°C.

## Revendications

1. Dispositif pour la mise en oeuvre d'une hydrogénation de benzène en cyclohexane avec participation d'une phase gazeuse contenant de l'hydrogène, d'une phase liquide contenant du benzène et d'une phase solide contenant du catalyseur, présentant
un organe de dispersion (6) qui est conçu pour la production d'un fluide réactionnel par dispersion d'une phase gazeuse dans une phase liquide,
au moins un réacteur (1) réalisé sous la forme d'un échangeur de chaleur avec entrée (31, 41) et sortie (43), le volume du réacteur étant limité par des parois dissipant la chaleur qui présentent un écart sensiblement constant le long de l'axe d'écoulement principal du fluide réactionnel et le réacteur étant équipé d'un treillis métallique (20, 32) enduit de catalyseur,
un conduit d'alimentation (7) qui conduit le fluide réactionnel de l'organe de dispersion (6) à l'entrée du réacteur (31, 41) et est suffisamment court pour que le degré de dispersité du fluide réactionnel ne se modifie pas sensiblement au cours du passage à travers le conduit d'alimentation.

2. Dispositif suivant la revendication 1, **caractérisé en ce que** le treillis métallique (20, 32) est un tissu métallique.

3. Dispositif suivant la revendication 1, **caractérisé en ce que** le treillis métallique (20, 32) est un tricot métallique.

4. Dispositif suivant l'une des revendications 1 à 3, **caractérisé en ce que** l'organe de dispersion (6) est un compresseur à gaz à jet de liquide.

5. Dispositif suivant l'une des revendications 1 à 4, **caractérisé en ce que** le réacteur (1) est réalisé sous la forme d'un échangeur de chaleur à plaques.

6. Dispositif suivant l'une des revendications 1 à 4, **caractérisé en ce que** le réacteur (1) est réalisé sous la forme d'un échangeur de chaleur en spirale.

7. Dispositif suivant l'une des revendications 1 à 6, **caractérisé en ce que** l'écart entre parois dans le réacteur est de 1 à 30 mm.

8. Dispositif suivant l'une des revendications 1 à 6, **caractérisé en ce que** l'écart entre parois dans le réacteur est de 2 à 20 mm.

9. Dispositif suivant l'une des revendications 1 à 6, **caractérisé en ce que** l'écart entre parois dans le réacteur est de 4 à 10 mm.

10. Procédé pour la mise en oeuvre d'une hydrogénation de benzène en cyclohexane avec participation d'une phase gazeuse contenant de l'hydrogène, d'une phase liquide contenant du benzène et d'une phase solide contenant du catalyseur, **caractérisé par** les étapes suivantes :
une production d'un fluide réactionnel par dispersion d'une phase gazeuse dans une phase liquide,
une traversée du fluide réactionnel produit au travers d'un réacteur dont le volume est équipé de tissus ou tricots métalliques qui ont été enduits de catalyseur,
un échange de la chaleur réactionnelle aux parois qui limitent le volume du réacteur, et
une séparation du fluide réactionnel en phase gazeuse et phase liquide.

11. Procédé suivant la revendication 10, **caractérisé par** le recyclage partiel, séparé, de phase gazeuse et/ou de phase liquide.

12. Procédé suivant la revendication 10, **caractérisé en ce que** la charge en liquide du réacteur est de 100 à 600 m³/(m² . h), par rapport à la section transversale du réacteur vide perpendiculairement à l'axe d'écoulement principal.

13. Procédé suivant la revendication 10, **caractérisé en ce que** la charge en gaz est de 0,5 à 15 cm/s, par rapport à la section transversale du réacteur vide- perpendiculairement à l'axe d'écoulement principal.

14. Procédé suivant la revendication 10, **caractérisé en ce que** le fluide réactionnel est dans le réacteur sous une pression de 0,1 à 200 bar.

15. Procédé suivant la revendication 10, **caractérisé en ce que** le fluide réactionnel présente dans le réacteur une température de 25 à 250 °C.
